# FASCICULE DE BREVET EUROPEEN

(11) **EP 3 999 154 B1**
(45) Date de publication et mention de la délivrance du brevet: **06.09.2023**
(21) Numéro de dépôt: 20753985.9
(22) Date de dépôt: 16.07.2020
(51) Int. Cl.: A61M 15/08

(54) **ENSEMBLE DE DISTRIBUTION DE PRODUIT FLUIDE**
ANORDNUNG FÜR DIE AUSGABE EINES FLÜSSIGPRODUKTS
ASSEMBLY FOR DISPENSING A FLUID PRODUCT

(30) Priorité: 19.07.2019 FR 1908249
(43) Date de publication de la demande: 25.05.2022
(73) Titulaire: APTAR France SAS, 27110 Le Neubourg (FR)
(72) Inventeur: ADAM, Fabien, 27930 AVIRON (FR); FARROCO, Vincent, 76630 ENVERMEU (FR)
(74) Mandataire: CAPRI
(86) Numéro de dépôt international: PCT/FR2020/051281
(87) Numéro de publication internationale: WO 2021/014076

(56) Documents cités:
- WO-A-98/57690
- WO-A1-02/085282
- WO-A2-2014/165694

## Description

La présente invention concerne un ensemble de distribution de produit fluide comportant un dispositif de distribution nasale de produit fluide et un appareil mobile distant, tel qu'une oreillette.

Les dispositifs de distribution nasale sont bien connus. Ils comportent généralement un réservoir contenant une ou plusieurs doses de produits fluide et une tête de distribution pour distribuer le produit fluide, notamment via une pompe, une valve doseuse ou un piston coulissant dans ledit réservoir. Lorsque l'utilisateur souhaite utiliser le dispositif, il insère la tête de distribution dans la narine et actionne le dispositif pour distribuer une dose de produit fluide, généralement sous forme de spray.

Un inconvénient avec les dispositifs de l'art antérieur concerne l'efficacité de la dose distribuée dans la narine, qui dépend souvent de l'orientation du dispositif au moment de la distribution de la dose. Par exemple, lorsque le produit fluide distribué a pour objectif d'agir sur le cerveau, seule une partie minime de la dose atteint généralement la zone cible pour ce type de traitement, à savoir la zone olfactive comprenant les éthmoïdes, notamment en raison d'une orientation du dispositif d'administration dans la narine qui est variable d'un patient à un autre. Or, il apparait que cette orientation conditionne la bonne visée de la zone cible, en particulier dans le cas d'un spray fermé utilisé pour obtenir le maximum de déposition dans la zone cible.

Le document WO9857690 décrit un dispositif de distribution nasale comportant un moyen d'orientation en appui sur la lèvre supérieure de l'utilisateur. S'il améliore la qualité de l'insertion, un tel dispositif d'orientation ne permet pas de garantir une orientation optimale au moment de la distribution de la dose.

Les documents WO02085282 et FR3024655 décrivent d'autres dispositifs de l'état de la technique.

La présente invention a pour but de fournir un ensemble de distribution qui ne reproduit pas les inconvénients susmentionnés.

La présente invention a notamment pour but de fournir un ensemble de distribution nasale permettant de maîtriser l'orientation du dispositif dans la narine, quelle que soit la morphologie du patient et quelle que soit sa position, debout, couché ou incliné, au moment de l'utilisation du dispositif.

La présente invention a aussi pour but de fournir un ensemble de distribution nasale qui améliore le taux de déposition de produit actif sur la zone olfactive et/ou les éthmoïdes.

La présente invention a également pour but de fournir un ensemble de distribution nasale qui permette de renseigner l'utilisateur en temps réel sur la qualité d'insertion du dispositif dans la narine.

La présente invention a encore pour but de fournir un ensemble de distribution nasale qui permette à l'utilisateur de corriger l'orientation du dispositif dans la narine au moment de son actionnement.

La présente invention a également pour but de fournir un ensemble de distribution nasale qui est simple et peu coûteux à fabriquer et à assembler.

La présente a donc pour objet un ensemble de distribution de produit fluide, comportant :
- un dispositif de distribution nasale de produit fluide, ledit dispositif nasal comportant un réservoir contenant du produit fluide, un organe de distribution pour distribuer une dose de produit fluide à chaque actionnement, et une tête de distribution assemblée sur ledit organe de distribution, ladite tête de distribution étant pourvue d'un orifice de distribution,
- un appareil mobile distant fixé sur la tête de l'utilisateur, tel qu'une oreillette,
dans lequel:
- ledit dispositif nasal et ledit appareil mobile distant fixé sur la tête de l'utilisateur comportent chacun un capteur d'orientation, tel qu'un accéléromètre ou un gyroscope, lesdits capteurs d'orientation coopérant pour déterminer la position du dispositif nasal dans une narine de l'utilisateur, ledit dispositif nasal comportant un indicateur, visible par l'utilisateur lorsque le dispositif nasal est inséré dans une narine, pour guider l'utilisateur vers une orientation optimale du dispositif nasal dans ladite narine, et
- ledit dispositif nasal comporte un module électronique et des moyens d'actionnement automatique adaptés à actionner automatiquement ledit dispositif nasal lorsque lesdits capteurs d'orientation détectent que ledit dispositif nasal est disposé dans une position optimale dans ladite narine.

Avantageusement, ledit dispositif nasal comporte un corps, comprenant un corps supérieur disposé autour dudit réservoir et dudit organe de distribution, et un corps inférieur, disposé sous ledit réservoir.

Avantageusement, ledit corps inférieur, de préférence amovible dudit corps supérieur, contient lesdits moyens d'actionnement automatique, notamment un élément poussoir et un moteur, adaptés lors de l'actionnement à déplacer ledit réservoir axialement vers le haut par rapport à ladite tête de distribution, pour ainsi actionner ledit organe de distribution.

Avantageusement, ledit indicateur comporte une tête d'indication lumineuse pourvue d'une pluralités d'indicateurs lumineux, tels que des diodes électroluminescentes, adaptées à assister et guider l'utilisateur en temps réel pour améliorer le positionnement dudit dispositif nasal.

Avantageusement, ledit indicateur est formé sur un bras solidaire, notamment de manière articulée, dudit dispositif nasal.

Avantageusement, ledit module électronique, tel qu'un circuit imprimé, comporte un microprocesseur contenant un logiciel de traitement des informations fournies par lesdits capteurs.

Avantageusement, ledit dispositif nasal comporte en outre un module de communication sans fil, avantageusement un module Bluetooth^{®}, pour une communication avec un appareil mobile distant, tel qu'un smartphone, une tablette ou un ordinateur.

Avantageusement, ledit dispositif nasal comprend un écran adapté à afficher des informations visibles par l'utilisateur.

Avantageusement, ledit dispositif nasal comprend un haut-parleur et/ou un élément vibrant, pour fournir une indication sonore et/ou tactile pour guider l'utilisateur lors du positionnement du dispositif nasal dans ladite narine.

Avantageusement, ledit indicateur comporte une caméra.

Avantageusement, ladite caméra détermine par reconnaissance faciale le positionnement spatial dudit dispositif nasal par rapport au visage, et donc par rapport à ladite narine.

Avantageusement, ledit dispositif nasal comporte un second capteur, tel qu'un capteur de luminosité, un capteur infrarouge, un capteur d'humidité, un capteur de température, pour détecter l'insertion dudit dispositif nasal dans ladite narine et/ou la profondeur d'insertion.

Avantageusement, ledit réservoir contient plusieurs doses de produit fluide, ledit organe de distribution étant une pompe ou une valve adaptée à distribuer une dose à chaque actionnement.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique d'un ensemble de distribution nasale selon un mode de réalisation avantageux,
les figures 2a et 2b sont des vues schématiques de l'ensemble de distribution de la figure 1, en cours d'utilisation par l'utilisateur, montrant l'indicateur visuel du dispositif qui informe l'utilisateur en temps réel de l'orientation verticale du dispositif dans la narine,
les figures 3a et 3b sont des vues similaires à celles des figures 2a et 2b, montrant l'orientation latérale du dispositif dans la narine,
la figure 4 est une vue schématique de face d'un visage, montrant les points de repères utilisés par une caméra dans une variante de réalisation avantageuse,
les figures 5 et 6 sont des vues schématiques d'un ensemble de distribution selon la variante de réalisation avantageuse avec caméra, en cours d'utilisation par l'utilisateur, montrant l'orientation verticale du dispositif dans la narine,
les figures 7 et 8 sont des vues schématiques de l'ensemble de la figure 1, en cours d'utilisation par l'utilisateur, montrant l'insertion du dispositif nasal dans la narine, et
les figures 9 et 10 sont des vues schématiques du dispositif nasal de la figure 1, respectivement avant et après actionnement.

La présente invention concerne plus particulièrement un ensemble de distribution avec un dispositif nasal multidoses, c'est-à-dire comportant un réservoir contenant plusieurs doses. Il est toutefois entendu que la présente invention ne se limite pas à ce type de dispositif, mais est au contraire applicable à tous les dispositifs de distribution de produit fluide et pulvérulents du type unidose, c'est-à-dire comportant un réservoir contenant une seule dose distribuée en un seul actionnement, bidose, c'est-à-dire comportant un réservoir contenant deux doses distribuées en deux actionnements successifs, ou multidoses, c'est-à-dire comportant un réservoir contenant plus de deux doses.

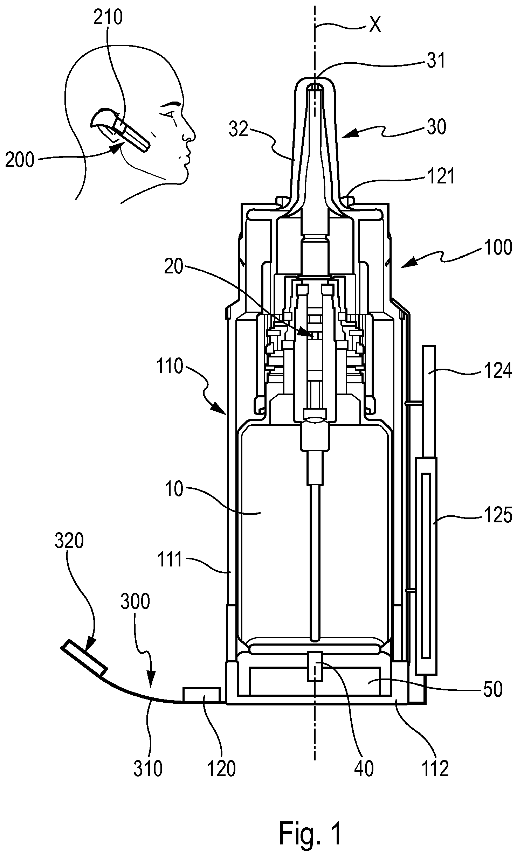

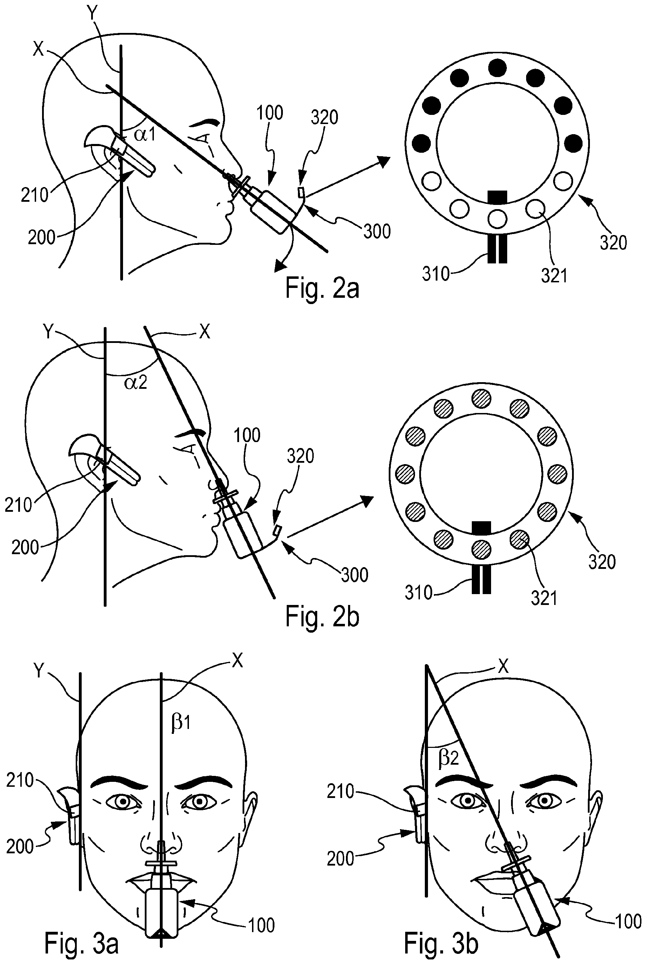

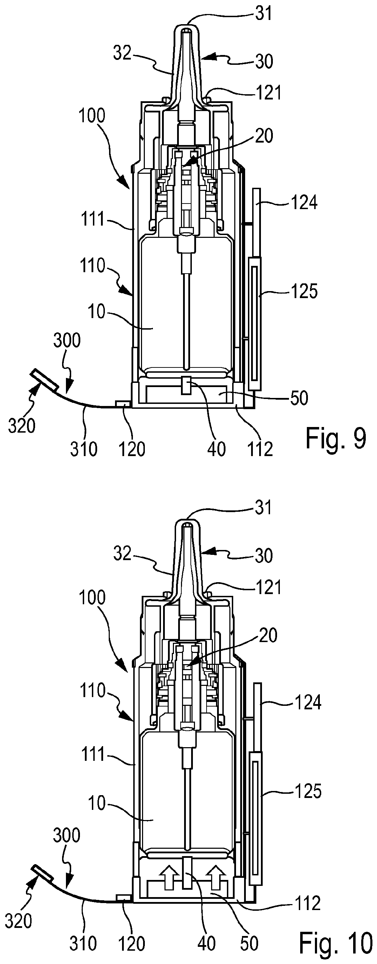

Dans la description ci-après, les termes "supérieur", "inférieur", "haut", "bas", "horizontal" et "vertical" se réfèrent à la position droite du dispositif représentée sur les figures 1, 9 et 10. Les termes "axial" et "radial" se réfèrent à l'axe central longitudinal X du dispositif, visible sur les figures 1 à 3b, 5 et 6.

Le dispositif nasal 100 donné à titre d'exemple est un multidose qui comporte un réservoir 10 contenant plusieurs doses de produit fluide, liquide ou pulvérulent. Un organe de distribution 20, tel qu'une pompe ou une valve, est assemblé sur le réservoir 10. Une tête de distribution 30 qui comporte un orifice de distribution 31 est assemblée sur ledit organe de distribution 20. La tête de distribution 30 comporte avantageusement un manchon creux 32, formant embout nasal, s'étendant axialement vers le haut et se terminant au niveau dudit orifice de distribution 31.

De préférence, le dispositif 100 comporte un corps 110. Celui-ci peut comporter un corps supérieur 111, disposé autour du réservoir 10 et de l'organe de distribution 20, et un corps inférieur 112, disposé sous ledit réservoir 10.

Le dispositif 100 comporte des moyens d'actionnement adaptés à déplacer le réservoir 10 par rapport à la tête de distribution 30, pour ainsi actionner l'organe de distribution 20.

Dans l'exemple représenté sur les figures, la tête de distribution 30 reste fixe par rapport au corps 110, et c'est le réservoir 10 qui est déplacé axialement vers le haut lors de l'actionnement par les moyens d'actionnement.

Avantageusement, l'actionnement du dispositif 100 est réalisé au moyen d'un élément poussoir 40 disposé dans le corps inférieur 112 et coopérant avec le fond du réservoir 10, pour déplacer ledit réservoir 10 axialement vers le haut par rapport à ladite tête de distribution 30. Un moteur 50 est prévu pour actionner ledit élément poussoir 40 lorsque le dispositif est dans la bonne orientation dans la narine, comme cela sera expliqué plus en détails ci-après. Avantageusement, le moteur 50 comporte une batterie rechargeable, par exemple au moyen d'une prise adaptée à cet effet. Avantageusement, le corps inférieur 112 est amovible, notamment pour simplifier la recharge de la batterie.

Le dispositif nasal 100 communique avec un appareil mobile distant 200 fixé sur la tête du patient, en particulier une oreillette, notamment via une communication sans fil ou filaire, afin d'assister et guider l'utilisateur en temps réel pour obtenir une orientation optimale du dispositif de distribution dans la narine au moment de son actionnement.

Un objectif est d'assurer un positionnement optimal du dispositif au moment même de l'actionnement du dispositif. Cela permet un meilleur taux de déposition du médicament dans la narine.

L'utilisateur peut éventuellement faire au préalable un test chez son spécialiste de santé afin de connaitre l'angle optimal adapté à sa morphologie, ce qui lui permet alors de configurer l'application associée. Eventuellement, on peut envisager l'utilisation d'un scanner ou d'une scintigraphie pour déterminer avec précision l'angle idéal.

Selon l'invention, le dispositif nasal 100 comporte un capteur d'orientation 120, tel qu'un accéléromètre ou un gyroscope, pour connaitre l'orientation spatiale du dispositif en temps réel, en particulier la position angulaire par rapport à l'appareil mobile distant 200.

L'appareil mobile distant 200, qui est fixé sur la tête de l'utilisateur, comporte également un capteur d'orientation 210, tel qu'un accéléromètre ou un gyroscope, permettant de déterminer sa position dans l'espace, notamment par rapport à la tête de l'utilisateur. Des moyens de communication appropriés sont prévus pour permettre à l'accéléromètre 210 de l'oreillette 200 de communiquer avec l'accéléromètre 120 du dispositif nasal 100.

Ainsi, les deux capteurs d'orientation 120 et 210 coopèrent pour déterminer avec précision la position du dispositif nasal dans la narine de l'utilisateur. En particulier, l'accéléromètre positionné sur le dispositif nasal 100 et l'accéléromètre de l'oreillette permettent d'obtenir en temps réel un positionnement spatial avec les différents angles en trois dimensions du dispositif nasal par rapport au patient. Plus particulièrement, par comparaison des angles entre lesdits capteurs d'orientation 120 et 210, on mesure l'angle d'inclinaison verticale α (c'est-à-dire vers le haut et le bas) et l'angle d'inclinaison latérale (c'est-à-dire vers la gauche ou la droite) entre l'axe X donné par le capteur d'orientation 120 du dispositif nasal 100 et un axe Y donné par le capteur d'orientation 210 de l'oreillette 200. L'orientation dudit axe Y par rapport à la narine de l'utilisateur est toujours identique, quelle que soit la position de l'utilisateur. Une mesure de la distance entre les deux capteurs d'orientation 120 et 210 permet en outre de déterminer la profondeur d'insertion P du dispositif nasal 100 dans la narine.

De préférence, le dispositif nasal 100 comporte un second capteur 121, tel qu'un capteur de luminosité, un capteur infrarouge, un capteur d'humidité, un capteur de température, pour détecter quand le dispositif 100 est inséré dans une narine. Ce second capteur 121 n'est pas obligatoire, mais il est avantageux pour identifier le moment où le dispositif nasal 100 est effectivement inséré dans la narine. Il peut dans ce cas déclencher l'électronique pour la faire passer d'un mode veille à un mode actif. Ce second capteur 121 peut être disposé en tout endroit approprié du dispositif nasal 100, par exemple en haut du corps 110, à la base du manchon 32 de la tête de distribution 30, comme visible sur les figures 1, 9 et 10. En variante, il pourrait aussi être disposé sur ledit manchon 32, par exemple à proximité de l'orifice de distribution 31. Par exemple, l'utilisation d'un capteur infrarouge permet de mesurer un temps aller/retour d'un signal. Ainsi plus le dispositif nasal est inséré dans la narine, plus ce temps est court. Ceci permet ainsi d'améliorer la précision de détermination de la profondeur d'insertion P.

Le dispositif nasal 100 comporte en outre un indicateur 300 pour le guider vers un positionnement optimal. Cet indicateur 300 est disposé de préférence sur un bras 310 qui permet de déporter l'indicateur latéralement par rapport au dispositif nasal afin d'être visible de l'utilisateur même lorsque le dispositif nasal est inséré dans la narine. Ce bras 310 peut être par exemple monté pivotant sur la corps inférieur 112, comme montré sur les dessins. Bien entendu, d'autres mises en oeuvre sont possibles.

Dans l'exemple des dessins, le capteur d'orientation 120 du dispositif nasal 100 est disposé sur ledit bras 310, mais il pourrait aussi être disposé à n'importe quel autre endroit du dispositif nasal 100.

L'indicateur 300 comporte une tête d'indication lumineuse 320, pourvue d'une pluralités d'indicateurs lumineux 321, tels que des diodes électroluminescentes (LED). Avantageusement, ces LEDs sont disposées en cercle pour former une couronne de LEDs. Ces LEDs peuvent s'illuminer dans différentes couleurs, pour indiquer à l'utilisateur comment modifier la position du dispositif nasal dans la narine. D'autres mises en oeuvre sont possibles, par exemples quatre indicateurs lumineux sous forme de flèche, orientés dans les quatre directions cardinales.

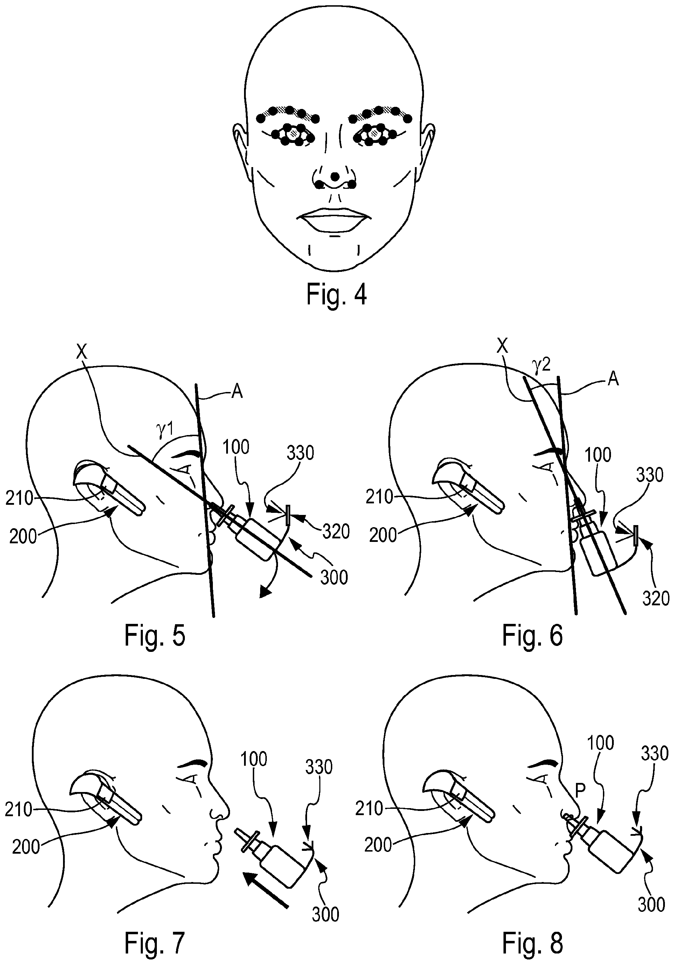

Dans une variante avantageuse, représentée sur les figures 4 à 8, la tête d'indication lumineuse 320 comporte une caméra 330, de préférence disposée au centre des indicateurs lumineux 321. Cette caméra 330 permet de déterminer le positionnement spatial du dispositif nasal 100 par rapport au visage, et donc par rapport à la narine. La caméra 330 permet ainsi d'appliquer en temps réel des points de repères, notamment autour des yeux, des sourcils et du nez du patient, comme illustré sur la figure 4, et de calculer des distances entre ces différents points de repère permettant de connaître la position du dispositif nasal 100 par rapport au visage et donc la narine du patient. Typiquement, la caméra 330 peut identifier plusieurs points de repère, par exemple vingt-cinq comme illustré sur la figure 4, qui montre trois repères sur le nez, six sur chaque oeil et cinq sur chaque sourcil. D'autres méthodes de reconnaissance faciale sont aussi envisageables. La caméra détermine ainsi l'angle α de l'axe longitudinal X du dispositif nasal 100 par rapport à un axe A du visage défini par lesdits points de repère. L'orientation dudit axe A par rapport à la narine de l'utilisateur est toujours identique, quelle que soit la position de l'utilisateur. Cet angle α détermine l'inclinaison verticale du dispositif nasal 100, c'est-à-dire vers le haut ou le bas. La caméra 330 permet aussi de déterminer l'angle d'inclinaison latérale β, c'est-à-dire vers la gauche ou la droite, ainsi que la profondeur d'insertion P.

Le dispositif nasal 100 comporte également un module électronique 125, tel qu'un circuit imprimé ou PCB, comportant un microprocesseur contenant le logiciel de traitement des informations fournies par le ou les capteurs, les moyens de commande du moteur d'actionnement 50 et éventuellement la caméra (si prévue). Le module électronique 125 peut aussi intégrer une fonction de blocage du dispositif pendant un temps prédéterminable après chaque actionnement, notamment si le produit fluide contenu dans le réservoir 10 est un médicament dangereux, du type fentanyl. Le module électronique peut avantageusement comporter en outre un module de communication, de préférence sans fil, avantageusement un module Bluetooth^{®}, pour une communication avec un second appareil mobile distant, tel qu'un ordinateur, un smartphone ou une tablette, pour transmettre des informations, par exemple l'horodatage de la dernière prise de dose, celui de la prochaine dose, la date de péremption du produit fluide, etc. Le module de communication permet aussi de surveiller l'utilisation du dispositif nasal, et par exemple d'alerter un tiers en cas de non utilisation du dispositif dans les délais requis.

Avantageusement, le dispositif 100 peut comporter un écran 124, notamment tactile, adapté à afficher des informations visibles par l'utilisateur. Il peut aussi comprendre un haut-parleur et/ou un élément vibrant, pour fournir une indication sonore et/ou tactile pour l'utilisateur, ce qui peut être utile pour des non-voyants par exemple.

Le fonctionnement de l'ensemble va être décrit ci-après en référence aux figures. Dans cet exemple, l'appareil mobile distant 200 est une oreillette.

Quelle que soit la position de l'utilisateur, debout, allongé ou assis, l'utilisateur place l'oreillette 200 sur son oreille et déploie le bras indicateur avant d'insérer le dispositif nasal 100 dans une narine.

Dans la variante avantageuse des figures 4 à 8, on utilise en outre la caméra 330 de l'indicateur 300, pour améliorer le positionnement du dispositif dans la narine. La figure 5 montre un angle γ1 non optimal, ici environ 60°, alors que la figure 6 montre un angle γ2 optimal, ici environ 30°.

Description des étapes de fonctionnement :
- Position repos : l'accéléromètre du dispositif nasal 100 n'indique aucun mouvement ; l'électronique 125 du dispositif est en veille ; l'application indique que le dispositif nasal 100 n'est pas dans la main du patient.
- Position première prise en main : l'accéléromètre 120 du dispositif nasal 100 détecte le mouvement du dispositif nasal ; l'électronique 125 du dispositif est activée; en variante, on peut utiliser le second capteur 121 optionnel pour activer l'électronique 125, comme expliqué précédemment, auquel cas cette activation ne se fait pas à la prise en main du dispositif nasal 100 mais lors de son insertion dans une narine.
- Position dispositif inséré dans la narine: la coopération des deux capteurs d'orientation 120 et 210 permet de déterminer la position du dispositif nasal 100 dans la narine, par comparaison des angles entre lesdits capteurs d'orientation ; dans la variante avec caméra 330, la mesure par reconnaissance faciale de l'angle d'inclinaison verticale γ, de l'angle d'inclinaison latérale (non représentée sur les dessins) et de la profondeur d'insertion P améliore encore davantage cette détermination de la position; si le positionnement est hors de la zone optimale, une indication, telle que des LEDs de couleur rouge, s'affiche sur l'indicateur 300; selon les LEDs qui s'allument dans la couronne d'indication, l'utilisateur sait quelle direction est mauvaise et va donc déplacer le dispositif en conséquence pour améliorer la position; parallèlement, des indications sonores peuvent être émises, telles que par exemple "inclinez le dispositif vers le haut/bas" et/ou "tournez le dispositif vers votre gauche/droite" ; de même, des vibrations du dispositif nasal peuvent aider l'utilisateur à améliorer le positionnement du dispositif nasal; lorsque l'utilisateur s'approche de l'orientation optimale, une indication différente, telle qu'une couleur différente pour les LEDs, peut s'afficher sur l'indicateur 300 et/ou les vibrations peuvent s'accélérer.

- Position optimale : lorsque l'utilisateur à positionné le dispositif nasal 100 dans la position optimale, toutes les LEDs s'affichent dans une couleur différente, par exemple en vert; les vibrations peuvent alors se transformer en un son continu; dans cette position optimale, l'électronique 125 actionne automatiquement le moteur 50 qui va pousser l'élément poussoir 40 et donc le réservoir 10 axialement vers le haut pour distribuer une dose de produit fluide ; parallèlement, une indication sonore peut être émise, telle que par exemple "actionnement du dispositif en cours"; ainsi, avant que la position optimale ne soit atteinte, le moteur 50 est inactif, et lorsque la position optimale est atteinte, le module électronique 125 enclenche le moteur 50 qui démarre l'administration de la dose de produit fluide.
- Après actionnement, l'écran 124 du dispositif 100, s'il est prévu, peut afficher l'horodatage de la dose qui a été émise, le nombre de doses restants à distribuer, l'horodatage de la prochaine dose, une indication de blocage du dispositif avec éventuellement un compte à rebours. D'autres indications sont aussi possibles, notamment si le dispositif 100 communique avec un appareil distant, tel qu'un ordinateur, une tablette ou un smartphone, pour transmettre des informations liées à l'utilisation du dispositif.

La présente invention procure ainsi de nombreux avantages :
- elle favorise un positionnement angulaire précis au moment de la distribution de dose, assurant ainsi un taux de déposition optimal du produit distribué dans la narine ;
- elle fournit une assistance "en temps réel" pour l'orientation ;
- elle assure une distribution automatique de la dose dès que la position optimale est atteinte, évitant ainsi tout risque de déplacement non souhaitable dû au geste d'actionnement; l'utilisateur n'a besoin que de tenir le dispositif nasal et de chercher la position optimale en fonction des indications de l'indicateur, l'actionnement de l'organe de distribution 20 étant géré automatiquement par les capteurs d'orientation 120, 210, l'électronique 125 et le moteur 50;

- elle fournit une solution utilisable avec un grand nombre de dispositifs de distribution nasale du même type, et n'est donc pas limitée à l'exemple décrit ;
- elle a un impact mineur sur la conception et l'encombrement du dispositif, et reste de ce fait facilement transportable ;
- elle n'a pas d'impact sur le fonctionnement et les performances du dispositif, et ne modifie donc pas les performances du produit fluide distribué ;
- elle permet d'administrer facilement le produit fluide à un tiers ;
- elle permet l'utilisation en toute position, notamment debout, assis, allongé ;
- elle permet un transfert de données aux médecins, spécialistes de santé, pharmaciens, réglementaire, assurances ;
- elle génère un auto-apprentissage : trouver l'angle optimal deviendra de plus en plus facile pour l'utilisateur, qui mémorisera inconsciemment la bonne position du dispositif par rapport à sa morphologie.

Bien entendu, la présente invention n'est pas limitée au dispositif multidoses décrit ci-dessus, mais s'applique à tout dispositif nasal, qu'il soit unidose, bidose ou multidoses.

La présente invention est notamment destinée au traitement de toute pathologie traitable par voie nasale nécessitant une orientation précise du dispositif nasal, tel que par exemple la maladie de Parkinson ou l'énurésie.

La présente invention a été décrite en référence à plusieurs modes de réalisation, mais il est entendu qu'un homme du métier peut y apporter toutes modifications, sans sortir du cadre de la présente invention tel que défini par les revendications annexées.

## Revendications

1. Ensemble de distribution de produit fluide, comportant :
- un dispositif de distribution nasale de produit fluide (100), ledit dispositif nasal (100) comportant un réservoir (10) contenant du produit fluide, un organe de distribution (20) pour distribuer une dose de produit fluide à chaque actionnement, et une tête de distribution (30) assemblée sur ledit organe de distribution (20), ladite tête de distribution (30) étant pourvue d'un orifice de distribution (31),
- un appareil mobile distant (200) adapté à être fixé sur la tête de l'utilisateur, tel qu'une oreillette,
**caractérisé en ce que**:
- ledit dispositif nasal (100) et ledit appareil mobile distant (200) adapté à être fixé sur la tête de l'utilisateur comportent chacun un capteur d'orientation (120; 210), tel qu'un accéléromètre ou un gyroscope, lesdits capteurs d'orientation (120; 210) coopérant pour déterminer la position du dispositif nasal (100) dans une narine de l'utilisateur, ledit dispositif nasal (100) comportant un indicateur (300), visible par l'utilisateur lorsque le dispositif nasal (100) est inséré dans une narine, pour guider l'utilisateur vers une orientation optimale du dispositif nasal (100) dans ladite narine, et
- ledit dispositif nasal (100) comporte un module électronique (125) et des moyens d'actionnement automatique (40; 50) adaptés à actionner automatiquement ledit dispositif nasal (100) lorsque lesdits capteurs d'orientation (120; 210) détectent que ledit dispositif nasal (100) est disposé dans une position optimale dans ladite narine.

2. Ensemble selon la revendication 1, dans lequel ledit dispositif nasal (100) comporte un corps (110), comprenant un corps supérieur (111) disposé autour dudit réservoir (10) et dudit organe de distribution (20), et un corps inférieur (112), disposé sous ledit réservoir (10).

3. Ensemble selon la revendication 2, dans lequel ledit corps inférieur (112), de préférence amovible dudit corps supérieur (111), contient lesdits moyens d'actionnement automatique, notamment un élément poussoir (40) et un moteur (50), adaptés lors de l'actionnement à déplacer ledit réservoir (10) axialement vers le haut par rapport à ladite tête de distribution (30), pour ainsi actionner ledit organe de distribution (20).

4. Ensemble selon l'une quelconque des revendications précédentes, dans lequel ledit indicateur (300) comporte une tête d'indication lumineuse (320) pourvue d'une pluralités d'indicateurs lumineux (321), tels que des diodes électroluminescentes, adaptées à assister et guider l'utilisateur en temps réel pour améliorer le positionnement dudit dispositif nasal (100).

5. Ensemble selon l'une quelconque des revendications précédentes, dans lequel ledit indicateur (300) est formé sur un bras (310) solidaire, notamment de manière articulée, dudit dispositif nasal (100).

6. Ensemble selon l'une quelconque des revendications précédentes, dans lequel ledit module électronique (125), tel qu'un circuit imprimé, comporte un microprocesseur contenant un logiciel de traitement des informations fournies par lesdits capteurs (120, 210).

7. Ensemble selon l'une quelconque des revendications précédentes, dans lequel ledit dispositif nasal (100) comporte en outre un module de communication sans fil, avantageusement un module Bluetooth^{®}, pour une communication avec un appareil mobile distant, tel qu'un smartphone, une tablette ou un ordinateur.

8. Ensemble selon l'une quelconque des revendications précédentes, dans lequel ledit dispositif nasal (100) comprend un écran (124) adapté à afficher des informations visibles par l'utilisateur.

9. Ensemble selon l'une quelconque des revendications précédentes, dans lequel ledit dispositif nasal (100) comprend un haut-parleur et/ou un élément vibrant, pour fournir une indication sonore et/ou tactile pour guider l'utilisateur lors du positionnement du dispositif nasal (100) dans ladite narine.

10. Ensemble selon l'une quelconque des revendications précédentes, dans lequel ledit indicateur (300) comporte une caméra (330).

11. Ensemble selon la revendication 10, dans lequel ladite caméra détermine par reconnaissance faciale le positionnement spatial dudit dispositif nasal (100) par rapport au visage, et donc par rapport à ladite narine.

12. Ensemble selon l'une quelconque des revendications précédentes, dans lequel ledit dispositif nasal (100) comporte un second capteur (121), tel qu'un capteur de luminosité, un capteur infrarouge, un capteur d'humidité, un capteur de température, pour détecter l'insertion dudit dispositif nasal (100) dans ladite narine et/ou la profondeur d'insertion.

13. Ensemble selon l'une quelconque des revendications précédentes, dans lequel ledit réservoir (10) contient plusieurs doses de produit fluide, ledit organe de distribution (20) étant une pompe ou une valve adaptée à distribuer une dose à chaque actionnement.

## Patentansprüche

1. Anordnung zur Abgabe eines Flüssigprodukts, umfassend:
- eine Vorrichtung (100) zur nasalen Abgabe eines Flüssigprodukts, wobei die nasale Abgabevorrichtung (100) einen Voratsbehälter (10) mit darin enthaltenem Flüssigprodukt, ein Abgabeelement (20) zur Abgabe einer Dosis Flüssigprodukt pro Betätigung und einen am Abgabeelement (20) angebrachten Abgabekopf (30) umfasst, wobei der Abgabekopf (30) mit einer Abgabeöffnung (31) versehen ist,
- ein zur Anbringung am Kopf des Benutzers geeignetes mobiles Remote-Endgerät (200), beispielsweise ein Headset, **dadurch gekennzeichnet, dass**
- die nasale Abgabevorrichtung (100) und das zur Anbringung am Kopf des Benutzers geeignete mobile Remote-Endgerät (200) jeweils einen Ausrichtungssensor (120; 210), beispielsweise einen Beschleunigungsmesser oder ein Gyroskop, umfassen, wobei die Ausrichtungssensoren (120; 210) zur Bestimmung der Position der nasalen Abgabevorrichtung (100) in einem Nasenloch des Benutzers zusammenwirken, wobei die nasale Abgabevorrichtung (100) eine für den Benutzer beim Einführen der nasalen Abgabevorrichtung (100) in ein Nasenloch sichtbare Anzeige (300) zur Benutzerführung im Hinblick auf die optimale Ausrichtung der nasalen Abgabevorrichtung (100) in dem Nasenloch aufweist, und
- die nasale Abgabevorrichtung (100) ein elektronisches Modul (125) sowie automatische Betätigungsmittel (40; 50) umfasst, die geeignet sind, eine automatische Betätigung der nasalen Abgabevorrichtung (100) zu bewirken, wenn mittels der Ausrichtungssensoren (120; 210) die optimale Positionierung der nasalen Abgabevorrichtung (100) in dem Nasenloch erkannt wird.

2. Anordnung nach Anspruch 1, bei dem die nasale Abgabevorrichtung (100) einen Körper (110) aufweist, der einen um den Vorratsbehälter (10) und das Abgabeelement (20) herum angeordneten oberen Körper (111) und einen unter dem Vorratsbehälter (10) angeordneten unteren Körper (112) umfasst.

3. Anordnung nach Anspruch 2, bei der der vorzugsweise von dem oberen Körper (111) abnehmbare untere Körper (112) die automatischen Betätigungsmittel, insbesondere ein Schieberelement (40) und einen Motor (50), enthält, die geeignet sind, bei ihrer Betätigung den Vorratsbehälter (10) in Bezug auf den Abgabekopf (30) axial nach oben zu verschieben und dadurch das Abgabeelement (20) zu betätigen.

4. Anordnung nach einem der vorhergehenden Ansprüche, bei der die Anzeige (300) einen Leuchtanzeigekopf (320) aufweist, der mit einer Mehrzahl von Leuchtanzeigen (321), zum Beispiel Leuchtdioden, versehen ist, die geeignet sind, den Benutzer in Echtzeit bei der Positionierung der nasalen Abgabevorrichtung (100) zu unterstützen und zu führen.

5. Anordnung nach einem der vorhergehenden Ansprüche, bei der die Anzeige (300) an einem Arm (310) ausgebildet ist, der einstückig, insbesondere gelenkig, mit der nasalen Abgabevorrichtung (100) verbunden ist.

6. Anordnung nach einem der vorhergehenden Ansprüche, bei der das elektronische Modul (125), beispielsweise eine gedruckte Schaltung, einen Mikroprozessor enthält, der eine Software zur Verarbeitung der von den Sensoren (120, 210) bereitgestellten Daten enthält.

7. Anordnung nach einem der vorhergehenden Ansprüche, bei der die nasale Abgabevorrichtung (100) des Weiteren ein drahtloses Kommunikationsmodul, vorzugsweise ein Bluetooth^{®}-Modul, zur Kommunikation mit einem mobilen Remote-Endgerät, beispielsweise einem Smartphone, einem Tablet oder einem Computer, umfasst.

8. Anordnung nach einem der vorhergehenden Ansprüche, bei der die nasale Abgabevorrichtung (100) ein Display (124) umfasst, das geeignet ist, um für den Benutzer sichtbar Daten anzuzeigen.

9. Anordnung nach einem der vorhergehenden Ansprüche, bei der die nasale Abgabevorrichtung (100) einen Lautsprecher und/oder ein Vibrationselement zur Abgabe einer akustischen und/oder taktilen Anzeige zur Benutzerführung bei der Positionierung der nasalen Abgabevorrichtung (100) in dem Nasenloch umfasst.

10. Anordnung nach einem der vorhergehenden Ansprüche, bei der die Anzeige (300) eine Kamera (330) umfasst.

11. Anordnung nach Anspruch 10, bei der die Kamera durch Gesichtserkennung die räumliche Positionierung der nasalen Abgabevorrichtung (100) in Bezug auf das Gesicht und damit in Bezug auf das Nasenloch bestimmt.

12. Anordnung nach einem der vorhergehenden Ansprüche, bei der die nasale Abgabevorrichtung (100) einen zweiten Sensor (121), beispielsweise einen Helligkeitssensor, einen Infrarotsensor, einen Feuchtigkeitssensor, einen Temperatursensor, zum Erfassen des Einführens der nasalen Abgabevorrichtung (100) in das Nasenloch und/oder der Einführtiefe aufweist.

13. Anordnung nach einem der vorhergehenden Ansprüche, bei der der Vorratsbehälter (10) mehrere Dosen Flüssigprodukt enthält, wobei es sich bei dem Abgabeelement (20) um eine Pumpe oder ein Ventil handelt, die bzw. das geeignet ist, um bei jeder Betätigung eine Dosis abzugeben.

## Claims

1. A fluid product dispensing assembly comprising:
- a device (100) for dispensing a fluid product nasally, said nasal device (100) comprising a reservoir (10) containing fluid product, a dispensing member (20) for dispensing a dose of fluid product upon each actuation, and a dispensing head (30) which is assembled on said dispensing member (20), said dispensing head (30) being provided with a dispensing orifice (31),
- a remote mobile device (200) such as an earpiece, adapted to be attached to the head of the user,
**characterized in that**:
- said nasal device (100) and said remote mobile device (200) adapted to be attached to the head of the user each comprise an orientation sensor (120; 210) such as an accelerometer or a gyroscope, said orientation sensors (120; 210) cooperating in order to determine the position of the nasal device (100) in a user's nostril, said nasal device (100) comprising an indicator (300) which is visible to the user when the nasal device (100) is inserted into a nostril in order to guide the user towards an optimal orientation of the nasal device (100) in said nostril, and
- said nasal device (100) comprises an electronic module (125) and automatic actuation means (40; 50) adapted to automatically actuate said nasal device (100) when said orientation sensors (120; 210) detect that said nasal device (100) is disposed in an optimal position in said nostril.

2. The assembly as claimed in claim 1, in which said nasal device (100) comprises a body (110) comprising an upper body (111) disposed around said reservoir (10) and said dispensing member (20), and a lower body (112) disposed under said reservoir (10).

3. The assembly as claimed in claim 2, in which said lower body (112), which is preferably removable from said upper body (111), contains said automatic actuation means, in particular a pusher element (40) and a motor (50), which are adapted, during actuation, to displace said reservoir (10) axially upwards with respect to said dispensing head (30) in order to actuate said dispensing member (20) thereby.

4. The assembly as claimed in any one of the preceding claims, in which said indicator (300) comprises a luminous indicator head (320) provided with a plurality of luminous indicators (321), such as light-emitting diodes, adapted to assist and guide the user in real time in order to improve the positioning of said nasal device (100).

5. The assembly as claimed in any one of the preceding claims, in which said indicator (300) is formed on an arm (310) which is integral with said nasal device (100), in particular in an articulated manner.

6. The assembly as claimed in any one of the preceding claims, in which said electronic module (125), such as a printed circuit, includes a microprocessor containing software for processing information provided by said sensors (120, 210).

7. The assembly as claimed in any one of the preceding claims, in which said nasal device (100) further comprises a wireless communication module, advantageously a Bluetooth^{®} module, for communication with a remote mobile device such as a smartphone, a tablet or a computer.

8. The assembly as claimed in any one of the preceding claims, in which said nasal device (100) comprises a screen (124) which is adapted to display information that can be seen by the user.

9. The assembly as claimed in any one of the preceding claims, in which said nasal device (100) comprises a loudspeaker and/or a vibrating element for providing an audible and/or tactile indication to guide the user when positioning the nasal device (100) in said nostril.

10. The assembly as claimed in any one of the preceding claims, in which said indicator (300) comprises a camera (330).

11. The assembly as claimed in claim 10, in which said camera determines, by means of facial recognition, the spatial positioning of said nasal device (100) with respect to the face, and thus with respect to said nostril.

12. The assembly as claimed in any one of the preceding claims, in which said nasal device (100) comprises a second sensor (121), such as a brightness sensor, an infrared sensor, a humidity sensor, a temperature sensor, in order to detect the insertion of said nasal device (100) into said nostril and/or to detect the depth of insertion.

13. The assembly as claimed in any one of the preceding claims, in which said reservoir (10) contains several doses of fluid product, said dispensing member (20) being a pump or a valve adapted to dispense one dose upon each actuation.
